(19) Europäisches Patentamt
European Patent Office
Office européen des brevets

(11) Veröffentlichungsnummer : **0 065 720**
**B1**

(12) # EUROPÄISCHE PATENTSCHRIFT

(45) Veröffentlichungstag der Patentschrift : **09.01.85**

(51) Int. Cl.$^4$ : **C 07 C 89/00**, C 07 C 91/44// C09B49/10

(21) Anmeldenummer : **82104220.7**

(22) Anmeldetag : **14.05.82**

(54) **Verfahren zur Herstellung von Hydroxydiphenylamin-Derivaten.**

(30) Priorität : **22.05.81 DE 3120387**

(43) Veröffentlichungstag der Anmeldung : **01.12.82 Patentblatt 82/48**

(45) Bekanntmachung des Hinweises auf die Patenterteilung : **09.01.85 Patentblatt 85/02**

(84) Benannte Vertragsstaaten : **CH DE FR GB IT LI**

(56) Entgegenhaltungen :
**EP-A- 0 011 254**
**DE-C- 46 869**
**JOURNAL OF THE CHEMICAL SOCIETY, 1952, London NG.PH. BUU-HOI "The Scope of the Knoevenagel Synthesis of Aromatic Secondary Amines" Seiten 4346 bis 4349**
**BEILSTEINS "Handbuch der organischen Chemie 4. Auflage, Band XIII, 1930, JULIUS SPRINGER VERLAG, Berlin Seite 444, Zeilen 10 bis 20**

(73) Patentinhaber : **CASSELLA Aktiengesellschaft**
**Hanauer Landstrasse 526**
**D-6000 Frankfurt am Main 61 (DE)**

(72) Erfinder : **Piesch, Steffen, Dr.**
**An der Heide 32**
**D-6370 Oberursel/Ts (DE)**
Erfinder : **Kemmerer, Hans**
**Lilienstrasse 4**
**D-6450 Hanau 8 (DE)**
Erfinder : **Weidemüller, Wolf, Dr.**
**Nordring 101**
**D-6000 Frankfurt 60 (DE)**
Erfinder : **Wille, Herbert, Dr.**
**Hünfelderstrasse 16**
**D-6000 Frankfurt 61 (DE)**

(74) Vertreter : **Urbach, Hans-Georg, Dr. et al**
**Hanauer Landstrasse 526**
**D-6000 Frankfurt am Main 61 (DE)**

**EP 0 065 720 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die vorliegende Erfindung betrifft ein Verfahren zur Herstellung von Hydroxydiphenylamin-Derivaten durch Kondensation von Dihydroxybenzol-Derivaten mit Anilin-Derivaten im Molverhältnis von 1 : 1 bis 1 : 10 bei erhöhter Temperatur, bei welchem die Kondensation bei 200 bis 330 °C in Gegenwart von 0,05 bis 2 Gew.% Jod, bezogen auf die Menge des Dihydroxybenzol-Derivats, als Katalysator durchgeführt, das Reaktionswasser durch Arbeiten im geschlossenen Gefäß im Reaktionssystem festgehalten und ggf. der Mischung der Ausgangsmaterialien vor Beginn der Reaktion zusätzlich Wasser oder ein wasserähnliches organisches Lösungsmittel zugesetzt wird.

Derivate des Hydroxydiphenylamins sind wertvolle Zwischenprodukte der chemischen Industrie und werden außerdem als Zusätze zu bestimmten Polymerisationsprozessen benötigt.

Eine besondere Bedeutung für die Herstellung von Schwefelfarbstoffen besitzt das p-Hydroxydiphenylamin. Durch Erhitzen dieser Verbindung mit wäßrigem Natriumpolysulfid 40 bis 50 Stunden auf 111 bis 112 °C unter Rückfluß wird der Schwefelfarbstoff C.I. Sulfur Red 10 (Brit. Patentschrift 753 764) und unter ähnlichen Schwefelungsbedingungen eine Reihe davon abgeleiteter brauner und rotbrauner Schwefelfarbstoffe hergestellt. Zur Herstellung dieser viel verwendeten, wertvollen Schwefelfarbstoffe muß das p-Hydroxydiphenylamin eine relativ hohe Reinheit besitzen. Verunreinigungen, insbesondere das in dem technischen Produkt häufig anzutreffende Diphenyl-p-phenylendiamin, machen das Produkt für die unmittelbare Herstellung des C.I. Sulfur Red 10 unbrauchbar, weil man beim Einsatz solcher verunreinigter Ausgangsmaterialien zu einem Schwefelfarbstoff gelangt, der sich nicht vorschriftsmäßig in Natriumsulfidlösung verküpen läßt, sondern der dabei einen schwarzen Rückstand ergibt, der, wenn er nicht vorher abfiltriert wird, die Färbungen verunreinigt und unbrauchbar macht. Das Filtrieren der Natriumsulfidküpe ist jedoch in Färbereien apparativ nicht vorgesehen und bringt herstellerseitig einen zusätzlichen Fertigungsaufwand und Farbstoffverluste mit sich. Auch zahlreiche Derivate des p-Hydroxydiphenylamins werden zur Herstellung von Schwefelfarbstoffen eingesetzt wie z. B. das 4-(β-Naphthylamino)-phenol, welches zur Herstellung von C.I. Sulphur Black 11 (US-PS 1 105 515) dient.

Es war daher stets ein technisches Problem, p-Hydroxy-diphenylamin-Derivate, insbesondere p-Hydroxydiphenylamin, selbst auf einfache Weise so herzustellen, daß es in der zur Schwefelung erforderlichen Reinheit in möglichst hoher Ausbeute erhalten wird.

In der Literatur sind zur Herstellung dieser Verbindung im wesentlichen vier verschiedene Wege beschrieben worden, nämlich :

1. Die sog. Indophenolreaktion, die auch in Spezialfällen Anwendung gefunden hat,
2. Die Umsetzung von p-Aminophenol mit Brombenzol in Gegenwart von Kupfer-I-jodid (Deutsche Patentschrift 187 870),
3. Die Umsetzung von p-Aminophenol-hydrochlorid mit Anilin (Deutsche Patentschrift 46 869) und
4. Die Umsetzung von Hydrochinon mit Anilin.

Von diesen 4 Verfahren ist nur das letzte Gegenstand häufiger Bearbeitungen und Verbesserungsvorschläge gewesen und hat sich in der Technik durchgesetzt.

Die grundlegenden Arbeiten zu diesem Verfahren wurden in Berichte der Deutschen Chemischen Gesellschaft, Bd. 16, S. 2790 ff von Arthur Calm veröffentlicht.

Nach den Angaben dieser Veröffentlichung läßt sich das p-Hydroxydiphenylamin dadurch herstellen, daß man Hydrochinon mit Anilin in einem Bombenrohr auf 290 bis 300 °C erhitzt. Eine solche Verfahrensweise ist naturgemäß für den großtechnischen Einsatz ungeeignet. Arbeitet man im vergrößerten Maßstab im Autoklaven unter den angegebenen Bedingungen, so erhält man beim Einsatz von 100 g Hydrochinon und 360 g Anilin 177 g eines Rohprodukts, das bei fraktionierter Destillation 86 g p-Hydroxydiphenylamin und ca. 80 g Diphenyl-paraphenylendiamin, neben 11 g Destillationsrückstand liefert. Die Ausbeute an p-Hydroxydiphenylamin beträgt nach diesem Verfahren somit ca. 51 % d. Th. Die Schwefelung des so hergestellten Rohprodukts führt zu einem Schwefelfarbstoff, der in Natriumsulfid-Lösung keine ausreichende Löslichkeit besitzt. Mit dem Ziel, die Ausbeute an p-Hydroxydiphenylamin zu erhöhen, ist dieses grundlegende Verfahren vielfach überarbeitet worden. Zunächst ist ein Jahr später vom gleichen Autor vorgeschlagen worden, zur Gewinnung etwas größerer Mengen dieser Substanz Hydrochinon mit dem Addukt aus Kalzium-chlorid und Anilin 10 Stunden auf 250 bis 260 °C zu erwärmen. Calm hat für dieses Verfahren keine Ausbeuten angegeben. Das rohe Reaktionsprodukt muß jedoch einer wäßrigen Reinigungsoperation unterzogen werden, um die große Menge des Kalziumchlorids zu entfernen.

Alle in der Folgezeit durchgeführten Überarbeitungen der Herstellung von Hydroxydiphenylamin sind im wesentlichen auf die Auswahl des sauren Katalysators und im Zusammenhang damit auf die Wahl der Arbeitstemperatur gerichtet, und allen diesen später bekanntgewordenen Verfahren ist das Abdestillieren des bei der Reaktion entstehenden Wassers gemeinsam. So betrifft die US-Patentschrift 2 156 792 aus dem Jahre 1939 die Umsetzung von Anilin mit Hydrochinon in Gegenwart von Zinkchlorid unter Abdestillieren des Reaktionswassers. Die US-Patentschrift 2 238 320 aus dem Jahre 1941 betrifft die Umsetzung von Anilin mit Hydrochinon in Gegenwart von Phosphorsäure unter Abdestillieren des Wassers. In Bios Report 1 (1954) S. 175 ist ein technisches Verfahren zur Umsetzung von Hydrochinon mit

Anilin angegeben, bei welchem als Katalysator Sulfaminsäure eingesetzt wird ; auch hierbei erfolgt ein kontinuierliches Abdestillieren des Reaktionswassers. Nach dem Verfahren der US-Patentschrift 3 450 746 aus dem Jahre 1969 und der DOS 28 50 391 vom 21.11.1978 verwendet man bei der Umsetzung von Dihydroxybenzolen mit Anilin-Derivaten p-Toluolsulfonsäure als Katalysator und destilliert das Reaktionswasser während der Reaktion kontinuierlich ab. Nach den Angaben der genannten US-Patentschrift werden 9 bis 18 % p-Toluolsulfonsäure, nach den Angaben der genannten DE-OS ca. 1 bis 10 Gew.% des sauren Katalysators zugesetzt. Zur Entfernung der Säure aus dem Endprodukt wird gemäß der genannten US-Patentschrift eine Wasser-Extraktion vorgenommen, während Kernstück der genannten DE-OS es ist, diese umständliche Reinigungsoperation dadurch zu vermeiden, daß das Reaktionsprodukt in Gegenwart einer Base, die zur Neutralisation des sauren Katalysators geeignet ist, im Vakuum fraktioniert destilliert wird.

Zusammenfassend ist somit festzustellen, daß Hydroxydiphenylaminderivate bislang im technischen Ausmaß stets durch Umsetzung von Dihydroxybenzol-Derivaten mit Anilin-Derivaten in Gegenwart saurer Kondensationsmittel, wie z. B. den Hydrochloriden oder Sulfaten der eingesetzten Anilinderivate, organischer Säuren, insbesondere Sulfonsäuren, $ZnCl_2$ oder anderen üblichen F.C. Katalysatoren hergestellt wurden. Wurden weniger als stöchiometrische Mengen der Kondensationsmittel verwendet, so wurde bisher immer das Wasser durch Destillation entfernt, wobei die Ausbeuten an 2- und 4-Hydroxyderivaten in der Regel 80 %, bezogen auf umgesetztes Dihydroxybenzol, nicht überschreiten und über den Verbleib der restlichen 20 % wenig geschrieben wird. Etwaige weitere mögliche Nebenprodukte außer den 1 : 2 Kondensationsprodukten wurden nicht identifiziert oder isoliert.

Aus Journal of Chem. Soc. London (1952) S. 4346 ff ist es bekannt, daß bei der Umsetzung von Hydrochinon mit Anilin oder Toluidin in Gegenwart von Jod in guter Ausbeute Bis-(Phenylamino)-benzol bzw. Bis-(Tolylamino)-benzol erhalten werden. Das Bis-(Phenylamino)-benzol (-N,N'-Diphenyl-p-phenylendiamin) entsteht, wie oben bereits ausgeführt, auch als hauptsächliches Nebenprodukt bei der Umsetzung von Hydrochinon mit Anilin in Gegenwart saurer Katalysatoren oder ohne Katalysator nach den Angaben des oben abgehandelten Standes der Technik und dürfte im wesentlichen verantwortlich sein für Schwierigkeiten, die bei der Herstellung von Schwefelfarbstoff aus technischem 4-Hydroxydiphenylamin immer wieder auftreten.

Es wurde nun überraschend gefunden, daß es möglich ist, bei der Kondensation von Dihydroxybenzolderivaten mit Anilinderivaten die Ausbeute an den gewünschten Hydroxydiphenylaminderivaten erheblich zu steigern und die Bildung der unerwünschten N,N'-Diphenyl-p-phenylendiaminderivate stark zurückzudrängen, wenn man bei der Kondensation der Di-hydroxybenzolderivate mit Anilinderivaten im Molverhältnis von 1 : 1 bis 1 : 10 bei einer Temperatur von 200 bis 330 °C in Gegenwart von 0,05 bis 2 Gew.% Jod, bezogen auf die Menge des Hydroxybenzolderivats, als Katalysator arbeitet und das Reaktionswasser durch druckfestes Verschließen des Reaktionsgefäßes im Reaktionssystem festhält. Darüber hinaus ist es in vielen Fällen vorteilhaft, der Mischung der Ausgangsmaterialien vor Beginn der Reaktion zusätzlich Wasser oder ein wasserähnliches organisches Lösungsmittel zuzusetzen, wobei der Zusatz von Wasser bevorzugt ist.

Besonders vorteilhaft ist das erfindungsgemäße Verfahren zur Herstellung von Hydroxydiphenylamin-Derivaten der Formel I

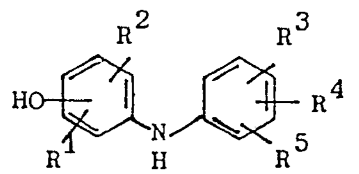

(I)

worin die —OH-Gruppe in 2-, 3- oder 4-Stellung stehen kann und $R^1$ bis $R^5$ unabhängig voneinander Wasserstoff, Alkyl mit 1 bis 6 C-Atomen, vorzugsweise 1 bis 3 C-Atomen, insbesondere Methyl oder Alkoxy mit 1 bis 4 C-Atomen, vorzugsweise 1 oder 2 C-Atomen, insbesondere Methoxy, bedeuten und einer von den Substituenten $R^3$, $R^4$ und $R^5$ auch eine Hydroxygruppe sein kann.

Zwei benachbarte Substituenten aus den Gruppen $R^1$ und $R^2$ und/oder $R^3$, $R^4$ und $R^5$ können auch direkt oder über ein Heteroatom zu einem ankondensierten 5- bis 7-gliedrigen Ring verbunden sein. Heteroatome, die als Brückenglied den Zusammenschluß zweier solcher Reste bewirken können, sind beispielsweise —O-, —S- und Stickstoff in Form der —NH-Gruppe. Der so gebildete 5- bis 7-gliedrige Ring kann seinerseits durch Alkyl mit 1 bis 4 C-Atomen, vorzugsweise 1 oder 2 C-Atomen, insbesondere Methyl substituiert sein. Vorzugsweise bedeutet einer der Substituenten $R^1$ oder $R^2$ Wasserstoff ; ferner ist es vorteilhaft, wenn von den 3 Substituenten $R^3$ bis $R^5$ mindestens einer ebenfalls Wasserstoff ist.

Man erhält diese, wenn man als Dihydroxybenzol-Derivat ein solches der Formel II

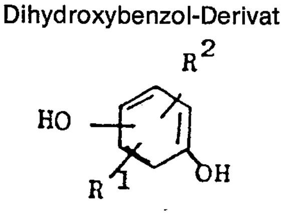

(II)

worin $R^1$ und $R^2$ die obengenannten Bedeutungen haben, und als Anilin-Derivat ein solches der Formel III

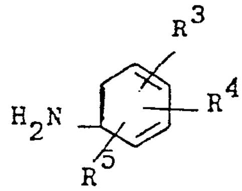

(III)

worin $R^3$, $R^4$ und $R^5$ die obengenannten Bedeutungen haben, einsetzt.

Die für $R^1$ bis $R^5$ stehenden Alkyl- und Alkoxyreste können geradkettig oder verzweigt sein.

Spezielle Vorteile bietet das erfindungsgemäße Verfahren zur Herstellung von solchen Hydroxydiphenylaminderivaten, bei denen die Hydroxygruppe in 2- oder 4-Stellung steht, da diese nach herkömmlichen Verfahren besonders geringe Ausbeuten liefern. Die größten wirtschaftlichen Vorteile ergeben sich bei der Herstellung der zur Schwefelfarbstoff-Herstellung besonders interessanten Verbindungen der Formel

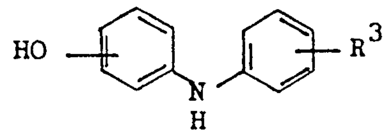

worin $R^3$ Wasserstoff, Methyl oder Methoxy ist.

Dihydroxybenzol-Derivate, die für die Umsetzung nach dem erfindungsgemäßen Verfahren in Betracht kommen, sind beispielsweise Hydrochinon, Resorcin, Brenzcatechin, Methylhydrochinon, 2,5-Dimethylhydrochinon, 2,3-Dimethylhydrochinon, 2,6-Dimethylhydrochinon, Ethylhydrochinon, 2,5- und 2,3-Diethylhydrochinon, Propylhydrochinon, 2,5-Dipropylhydrochinon, Butylhydrochinon, Pentylhydrochinon, Hexylhydrochinon, 4- und 5-Methylresorcin, 4- und 5-Ethylresorcin, 4- und 5-Propylresorcin, 4- und 5-Butylresorcin, 4- und 5-Pentylresorcin, 4- und 5-(n)-Hexylresorcin, 4- und 5-Isohexylresorcin, 2,4-, 2,5-, 4,5- und 4,6-Dimethylresorcin, 2,4-, 2,5-, 4,5- und 4,6-Diethylresorcin, 4,5- und 4,6-Dipropylresorcin, 4,5- und 4,6-Dibutylresorcin, 4,5- und 4,6-Diisobutylresorcin, 4,5- und 4,6-Di-tert.-butylresorcin, 3- und 4-Methylbrenzcatechin, 3- und 4-Ethylbrenzcatechin, 3- und 4-Propyl- oder Isopropylbrenzcatechin, 3- und 4-(n)-Butyl-, Isobutyl-, (sec.) Butyl- oder (tert.) Butylbrenzcatechin, 3- und 4-Pentylbrenzcatechin, 3- und 4-(n)-Hexyl-brenzcatechin, 3,5- und 4,5-Dimethylbrenzcatechin, 3,5- und 4,5-Diethylbrenzcatechin, 3,5- und 4,5-Dipropylbrenzcatechin, 3,4- und 4,5-Di-(n)-butylbrenzcatechin, Di-(tert.)-butylbrenzcatechin, Methoxy-, Ethoxy-, Propoxy- und Butoxyhydrochinon, 4- oder 5-Methoxyresorcin, 4- oder 5-Ethoxyresorcin, 4- oder 5-Butoxyresorcin, 3- oder 4-Methoxy-, Ethoxy-, Propoxy- und Butoxybrenzcatechin, 2,5-Dihydroxy-3-methoxy-toluol, ethylbenzol, -propyl- bzw. isopropylbenzol, -(n)-butyl, -isobutyl-, -(tert.)-butylbenzol und -pentylbenzol, 2,5-Dihydroxy-3-ethoxy-, -propoxy-, -isopropoxy-, -butoxy und -(tert.)-butoxy-toluol, 3,6-Dihydroxy-2-methoxy-toluol, 2,5-Dihydroxy-4-methoxytoluol, 2,6-Dihydroxy-4-methoxytoluol, 2,4-Dihydroxy-6-methoxy-toluol, 2,4-Dihydroxy-4-ethoxy-, -propoxy- und -butoxy-toluol, 2,5-Dihydroxy-4-ethoxy-ethylbenzol, 2,5-Dihydroxy-4-(n)-butoxy-propylbenzol, 2,5-Dimethoxyhydrochinon, 2,5-Diethoxyhydrochinon, 2,5-Diisopropoxyhydrochinon, 2,5-Di-(n)-butoxyhydrochinon, 2,5-Di-(tert.)-butoxyhydrochinon, 2,6-Dimethoxy- und -Diethoxyhydrochinon, 3,4- und 2,5-Dimethoxyresorcin, 3,4-Diethoxyresorcin, 3,4-, 3,5- und 3,6-Dimethoxybrenzcatechin, 1,4-Dihydroxynaphthalin, 2,3-Dihydroxynaphthalin, Methyl-1,4-dihydroxynaphthalin, Methyl-2,3-dihydroxynaphthalin.

Anilin-Derivate, die für den Einsatz in dem erfindungsgemäßen Verfahren in Betracht kommen, sind beispielsweise Anilin, o-, m- oder p-Toluidin, 2,3-, 2,4-, 2,5-, 2,6-, 3,4- oder 3,5-Dimethylanilin, 2,3,4-, 2,3,5-, 2,3,6-, 2,4,5-, 2,4,6- oder 3,4,5-Trimethylanilin, o-, m- und p-Ethylanilin, o-, m- und p-Propyl- und Isopropylanilin, o- und p-(n)-Butylanilin, o- und p-(tert.)-Butylanilin, o- und p-(n)-Hexyl- und -Isohexylanilin, 2,4-Diethylanilin, 2,6-Diisopropylanilin, 2,4-Dibutylanilin, 2,4,6-Tri-(tert.)-butylanilin, o-, m- und p-Anisidin, o-, m- und p-Phenetidin, 2- und 4-Butoxyanilin, 2,4-, 2,5- und 3,5-Dimethoxyanilin, 2,4-Dibutoxyanilin, 3,4,5-Trimethoxyanilin, 3,4,5-Triethoxyanilin, 2-Methoxy-5-methylanilin, 4-Methoxy-, -Ethoxy, Propoxy- oder -Butoxy-2-methylanilin, 2-Methoxy-4-methylanilin, 2-Methoxy-4-ethylanilin, 2-Methoxy-4-isopropylanilin, 2-Methoxy-4-butylanilin, 2-Methoxy-4-(tert.)-butylanilin, 2-Methoxy-4-hexylanilin, 2-Methoxy-4,5-dimethylanilin, α- oder β-Aminonaphthalin, Methyl-α- oder β-Aminonaphthalin, Aminotetralin, o-, m- oder p-Butylanilin, o-, m- oder p-Hydroxyanilin.

Besonders günstig lassen sich nach dem erfindungsgemäßen Verfahren Hydrochinon, Brenzcatechin, Resorcin als Dihydroxybenzol-Komponente und Anilin, o-, m- und p-Toluidin, Xylidine, o-, m- und p-Anisidin, o-, m- und p-Phenetidin und 2,4-Dimethoxyanilin als Anilin-Komponente umsetzen.

Die Ausgangsmaterialien werden in einem druckfest verschlossenen Kessel auf die Reaktionstemperatur erwärmt, wobei in der Regel für eine ständige Durchmischung des Ansatzes, z. B. durch Rühren gesorgt wird. Durch die Anwesenheit des Wassers, welches ggf. erst im Verlauf der Reaktion gebildet

wird, ergibt sich ein Innendruck von 10 bis 60 bar, je nach der im Bereich von 200-330 °C gewählten Reaktionstemperatur. Vorzugsweise arbeitet man bei einer Innentemperatur von 220-270 °C, wobei in der Regel ein Innendruck von 20 bis 40 bar entsteht. Der Jodzusatz beträgt vorzugsweise 0,1 bis 1 Gew.%, bezogen auf das Gewicht des eingesetzten Dihydroxybenzol-Derivats.

Wird ohne anfänglichen Wasserzusatz gearbeitet, so baut sich im Verlauf der Reaktion der Innendruck in dem Maße auf, wie das Reaktionswasser gemäß dem Formelschema

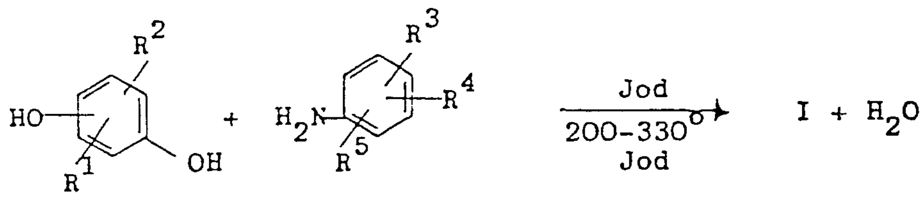


freigesetzt wird.

Sofern vor Beginn der Reaktion Wasser zugesetzt wird, wie es insbesondere bei Einsatz von Hydrochinon und Hydrochinon-Derivaten zweckmäßig ist, wird von Anfang an ein relativ hoher Innendruck erreicht.

Die gegebenenfalls zugesetzte Wassermenge beträgt bis zu 3 Mol pro Mol des Dihydroxybenzol-Derivats. Vorzugsweise werden 0,5 bis 1,5 Mol Wasser zugesetzt.

Ein ähnlich vorteilhafter Effekt wie der durch Wasserzusatz erzielbare wird bei Zusatz eines wasserähnlichen organischen Lösungsmittels erhalten. Hiervon werden normalerweise bis zu 10 Gew.% bezogen auf das Gesamtgewicht der Ausgangsmaterialien zugesetzt. Als wasserähnliches Lösungsmittel wird vorwiegend ein Glykol oder Polyäther der Formel

$$HO-(CH_2\underset{R}{CH}-O)_nH$$

eingesetzt, wobei R Wasserstoff oder Methyl ist und n eine Zahl von 1 bis 10 bedeutet, oder ein Monomethyl- oder Mono-ethyläther eines solchen Polyäthers oder auch ein Alkanol mit 1 bis 4 C-Atomen.

Die Reaktion dauert maximal 40 Stunden, in den meisten Fällen jedoch nur 2 bis 8 Stunden. Der Fortschritt und das Ende der Reaktion lassen sich recht gut am Aufbau des Innendruckes verfolgen. Das Reaktionsende ist erreicht, wenn der Druck nicht mehr weiter steigt; ein Überschreiten der erforderlichen Reaktionszeit ist jedoch praktisch ohne Einfluß auf die Ausbeute.

Das Reaktionsgeschehen sei im folgenden auf das Beispiel der Umsetzung von Hydrochinon mit Anilin bezogenen Reaktionsschema veranschaulicht

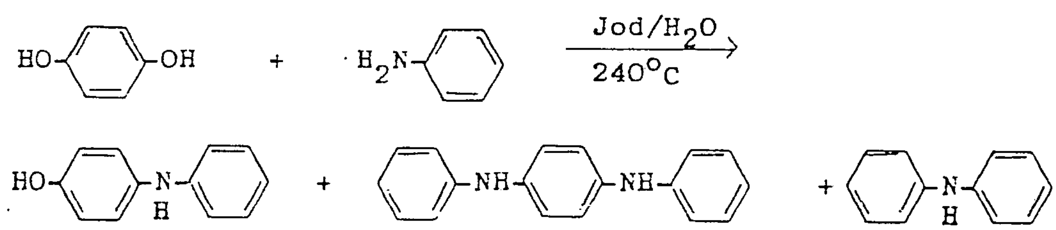


(Ia) ~ 91 % d. Th. — (II) ~ 6 bis 8 % d. Th. — ~ ca. 1-2 %

+ undestillierbare Polykondensationsprodukte.

Neben der sehr erwünschten Erhöhung der Ausbeute an Endprodukt der Formel I ergibt sich noch bei der Durchführung des erfindungsgemäßen Verfahrens ein zusätzlicher Vorteil dadurch, daß keine sauren Katalysatoren eingesetzt werden, wodurch die Aufarbeitung des rohen Reaktionsprodukts ohne weiteres durch eine einfache Vakuumdestillation erfolgen kann und somit wesentlich erleichtert wird. Die Isolierung des Nebenproduktes der Formel II gemäß vorstehendem Formelschema in reiner Form bereitet keine Schwierigkeiten, und die Menge des undestillierbaren Rückstands wird auf ein Minimum beschränkt.

Da beim erfindungsgemäßen Verfahren, im Gegensatz zu den bisher bekannten, das Dihydroxybenzol-Derivat praktisch quantitativ umgesetzt wird, ist es nicht erforderlich, dieses zu regenerieren.

Die folgenden Ausführungsbeispiele veranschaulichen die vorliegende Erfindung.

## Beispiel 1

In einem 3 l-Edelstahlautoklaven werden 1,4 kg Anilin, 550 g Hydrochinon, 90 g Wasser und 3 g Jod 12 Stunden bei 260 °C gerührt. Nach ca. 6 Stunden stellt sich ein maximaler Innendruck von 34 bar ein. Nach dem Öffnen des Autoklaven wird der Inhalt entnommen und fraktioniert destilliert. Zunächst wird ein Vorlauf abdestilliert, bis bei 15 mbar eine Übergangstemperatur von 90 °C erreicht ist. Der Vorlauf besteht im wesentlichen aus Wasser, dem überschüssigen Anilin und etwas Diphenylamin. Das Hauptprodukt, 4-Hydroxydiphenylamin, destilliert dann bei einem Druck von 0,4 bis 0,7 mbar bei 155-170 °C. Man erhält davon 845 g, entsprechend 91,4 % d. Th. Anschließend läßt sich bei 0,2 mbar und 225-235 °C noch das in geringer Menge entstandene 4,4'-Diphenyl-p-phenylendiamin abdestillieren. Hiervon erhält man 90 g entsprechend ca. 7 % d. Th. In der Destillationsblase verbleiben 15 bis 20 g undestillierbarer Rückstand.

## Beispiel 2

In einem 3 l-Edelstahlautoklaven werden 1 260 g Anilin, 500 g Hydrochinon, 2,5 g Jod und 85 g Wasser 16 Stunden auf 240 °C erwärmt, wobei der Innendruck gleichförmig bis auf 23 bar ansteigt. Nach Öffnen des Autoklaven wird der Inhalt wie im Beispiel 1 beschrieben fraktioniert destilliert. Nach Abnahme des Vorlaufs destilliert bei einem Druck von 5 mbar bei 185 °C beginnend und bei 0,2 mbar bei 177 °C endend dann das Hauptprodukt. Die Ausbeute beträgt 750,1 g entsprechend 89,2 % d. Th.

Bei 0,1 bis 0,2 mbar destillieren bei 192 bis 215 °C noch 79,2 g (~ 6,7 % d. Th.) 4,4'-Diphenyl-p-phenylendiamin über.

## Beispiel 3

In einem 1,5 l-Edelstahlautoklaven werden 450 g m-Aminophenol, 300 g Resorcin, 30 g Wasser und 2 g Jod 20 Stunden bei 220 °C gerührt. Nach ca. 8 Stunden stellt sich ein maximaler Innendruck von 12 bar ein. Nach dem Öffnen des Autoklaven wird der Inhalt entnommen und fraktioniert destilliert. Zunächst wird ein Vorlauf abdestilliert, bis bei 15 mbar eine Übergangstemperatur von 90 °C erreicht ist. Dieser Vorlauf besteht im wesentlichen aus Wasser. Als zweite Fraktion, die von 128 °C bei 1 mbar bis 145 °C bei 0,2 mbar destilliert, wird überschüssiges m-Aminophenol erhalten. Das Hauptprodukt, 3,3'-Dihydroxydiphenylamin, destilliert dann bei einem Druck von 0,8 bis 1,0 mbar bei 210 bis 240 °C, wobei die Hauptmenge bei 0,9-1 mbar bei 225-230 °C überdestilliert. Man erhält davon 455 g, entsprechend 83 % d. Th. Der Schmelzpunkt des so erhaltenen 3,3'-Dihydroxydiphenylamins ist 140 °C.

Führt man den gleichen Ansatz drucklos am Rückflußkühler aus, wobei 40 Std. bei 200 °C gerührt wird, so erhält man bei der gleichen Aufarbeitung 334 g 3,3'-Dihydroxydiphenylamin entsprechend ca. 61 % d. Th.

Analog den obigen Ausführungsbeispielen lassen sich auch die Beispiele der folgenden Tabelle durchführen.

Die Tabelle bezieht sich auf die Umsetzung von Dihydroxybenzolen mit Anilin und Anilinderivaten. Die Ausbeute betrifft das gewünschte, entsprechende Hydroxydiphenylamin bzw. Hydroxydiphenylaminderivat und bezieht sich auf die Menge des eingesetzten Dihydroxybenzols.

Das in der dritten Spalte angegebene Molverhältnis ist das von Anilin-Derivat : Dihydroxybenzol. Die angegebenen Zusatzmengen von Wasser (in Mol) und/oder wasserähnlichem Lösungsmittel (in %) sowie von Jod (in %) beziehen sich auf die Menge des eingesetzten Dihydroxybenzols.

(Siehe Tabelle Seite 7 ff.)

Tabelle

| Dihydroxybenzol | Anilinderivat | Molverhältnis | Wasser bzw. wasserähnl. Lösungsm. | Jod [%] | Reaktionsdauer [h] Temp. [°C] Druck [bar] | Ausbeute |
|---|---|---|---|---|---|---|
| Hydrochinon | Anilin | 3 : 1 | $H_2O$ 1 Mol | 0,54 | 12 h 260°C 34 bar | 91 % |
| " | " | 3 : 1 | Polydiol 300 16 % | 0,54 | 12 h 260°C 29 bar | 89 % |
| " | " | 3 : 1 | $H_2O$ 1 Mol | 0,54 | 12 h 240°C 22 bar | 86 % |
| " | " | 2 : 1 | - | 0,67 | 4 h 260°C 23 bar | 83 % |
| " | " | 5 : 1 | $H_2O$ 0,5 Mol Polydiol 300 5 % | 0,5 | 12 h 260°C 29 bar | 88 % |
| Resorcin | Anilin | 3 : 1 | - | 0,54 | 8 h 260°C 24 bar | 90 % |

Tabelle (Fortsetzung)

| Dihydroxy-benzol | Anilin-derivat | Molverhältnis | Wasser bzw. wasserähnl. Lösungsm. | Jod [%] | Reaktionsdauer [h]<br>Temp. [°C]<br>Druck [bar] | Ausbeute |
|---|---|---|---|---|---|---|
| Resorcin | o-Toluidin | 3 : 1 | - | 0,54 | 12 h<br>260°C<br>23 bar | 95 % |
| Brenzkatechin | Anilin | 3 : 1 | $H_2O$ 1 Mol | 0,54 | 6 h<br>260°C<br>20 bar | 78 % |
| | Vergleichsversuch gemäß A. Calm Ber. Bd 16, S.2790 ff | | | | | |
| Hydrochinon | Anilin | 4,25 : 1 | - | - | 16 h<br>295°C | 51 % |

**Ansprüche**

1. Verfahren zur Herstellung von Hydroxydiphenylamin-Derivaten durch Kondensation von Dihydroxybenzol-Derivaten mit Anilin-Derivaten im Molverhältnis von 1 : 1 bis 1 : 10 bei erhöhter Temperatur, dadurch gekennzeichnet, daß die Kondensation bei 200 bis 330 °C in Gegenwart von 0,05 bis 2 Gew.% Jod, bezogen auf die Menge des Dihydroxybenzol-Derivats als Katalysator durchgeführt, das Reaktionswasser durch Arbeiten im geschlossenen Gefäß im Reaktionssystem festgehalten und ggf. der Mischung der Ausgangsmaterialien vor Beginn der Reaktion zusätzlich Wasser oder ein Glykol oder Polyäther der Formel

$$HO-(CH_2\underset{R}{CH}-O)_nH$$

wobei R Wasserstoff oder Methyl ist und n eine Zahl von 1 bis 10 bedeutet, oder ein Monomethyl- oder Mono-ethyläther eines solchen Polyäthers oder auch ein Alkanol mit 1 bis 4 C-Atomen zugesetzt wird.

2. Verfahren gemäß Anspruch 1 zur Herstellung von Hydroxydiphenylamin-Derivaten der Formel I

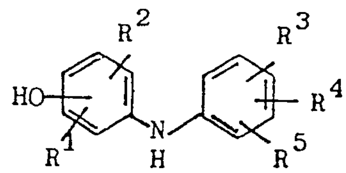

(I)

worin die —OH-Gruppe in 2, 3 oder 4-Stellung stehen kann und $R^1$ bis $R^5$ unabhängig voneinander Wasserstoff, Alkyl mit 1 bis 6 C-Atomen oder Alkoxy mit 1 bis 4 C-Atomen bedeuten und einer von den Substituenten $R^3$, $R^4$ und $R^5$ auch eine Hydroxygruppe sein kann, wobei zwei benachbarte Substituenten aus den Gruppen $R^1$ und $R^2$ und/oder $R^3$, $R^4$ und $R^5$ auch direkt oder über ein Heteroatom zu einem ankondensierten 5 bis 7 gliedrigen Ring verbunden sein können und der so gebildete 5 bis 7 gliedrige Ring, seinerseits durch Alkyl mit 1 bis 4 C-Atomen substituiert sein kann, dadurch gekennzeichnet, daß man als Dihydroxybenzol-Derivat ein solches der Formel II

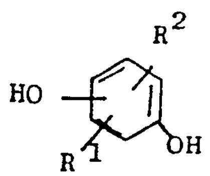

(II)

worin $R^1$ und $R^2$ die obengenannten Bedeutungen haben, und als Anilin-Derivat ein solches der Formel III

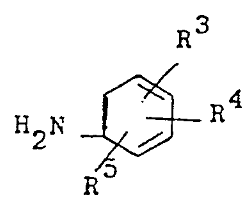

worin $R^3$, $R^4$ und $R^5$ die obengenannten Bedeutungen haben einsetzt.

3. Verfahren nach den Ansprüchen 1 und 2, dadurch gekennzeichnet, daß bei 220 bis 270 °C kondensiert wird.

4. Verfahren nach den Ansprüchen 1 bis 3, dadurch gekennzeichnet, daß 0,1 bis 1 Gew.% Jod als Katalysator eingesetzt werden.

5. Verfahren nach den Ansprüchen 1 bis 4, dadurch gekennzeichnet, daß der Mischung der Ausgangsmaterialien vor der Reaktion bis zu 3 Mol Wasser pro Mol des Dihydroxybenzol-Derivats zugesetzt werden.

6. Verwendung der gemäß den Ansprüchen 1 bis 5 hergestellten Hydroxydiphenylamin-Derivate zur Herstellung von Schwefelfarbstoffen.

**Claims**

1. Process for the manufacture of hydroxydiphenylamine derivatives by subjecting dihydroxybenzene derivatives to a condensation reaction with aniline derivatives in a molar ratio of 1 : 1 to 1 : 10 at elevated temperature, characterised in that the condensation reaction is carried out at 200 to 330 °C in the presence, as a catalyst of 0.05 to 2 % by weight of iodine, relative to the quantity of the dihydroxybenzene derivative, the water of reaction is retained within the reaction system by carrying out the reaction in a closed vessel and, if appropriate there is added to the mixture of the starting materials before the commencement of the reaction, further water or a glycol or polyether of the formula

$$HO-(CH_2\underset{R}{CH}-O)_nH$$

where R denotes hydrogen or methyl and n is a number from 1 to 10, or a monomethyl ether or monoethyl ether of such a polyether or an alkanol having 1 to 4 carbon atoms.

2. Process according to Claim 1 for the manufacture of hydroxydiphenylamine derivatives of the formula I

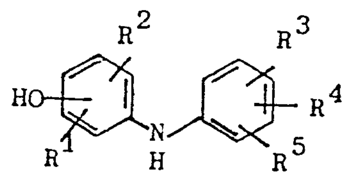

(I)

where the —OH group can be in the 2-, 3- or 4-position and $R^1$ to $R^5$ independently of one another denote hydrogen, alkyl having 1 to 6 C atoms or alkoxy having 1 to 4 C atoms, and one of the substituents $R^3$, $R^4$ and $R^5$ may also be a hydroxyl group, it being also possible for two adjacent substituents from the groups $R^1$ and $R^2$ and/or $R^3$, $R^4$ and $R^5$ to be attached direct or via a hetero-atom to form a fused 5-membered to 7-membered ring and for the 5-membered to 7-membered ring thus formed in turn to be substituted by alkyl having 1 to 4 C atoms, characterised in that the dihydroxybenzene derivative employed is a derivative of the formula II

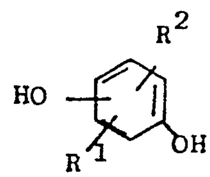

(II)

where $R^1$ and $R^2$ have the abovementioned meanings, and the aniline derivative employed is a derivative of the formula III

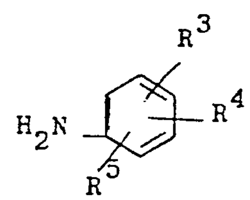

(III)

where $R^3$, $R^4$ and $R^5$ have the abovementioned meanings.

3. Process according to Claims 1 and 2, characterised in that the condensation reaction is carried out at 220 to 270 °C.

4. Process according to Claims 1 to 3, characterised in that 0.1 to 1 % by weight of iodine is employed as the catalyst.

5. Process according to Claims 1 to 4, characterised in that up to 3 mols of water per mol of the dihydroxybenzene derivative are added to the mixture of the starting materials before the reaction.

6. Use of the hydroxydiphenylamine derivatives prepared in accordance with Claims 1 to 5 for the manufacture of sulphur dyestuffs.

**Revendications**

1. Procédé de préparation de dérivés d'hydroxydiphénylamines par condensation de dérivés de dihydroxybenzènes avec des dérivés de l'aniline dans un rapport molaire compris entre 1 : 1 et 1 : 10, à chaud, procédé caractérisé en ce qu'on effectue la condensation entre 200 et 330 °C en présence comme

catalyseur de 0,05 à 2 % en poids d'iode par rapport à la quantité du dérivé de dihydroxybenzène, on maintient dans le système réactionnel l'eau formée par la réaction en opérant dans un récipient fermé, et le cas échéant, avant de commencer la réaction, on ajoute en plus au mélange des matières dont on part de l'eau ou un glycol ou un polyéther de formule

$$HO-(CH_2-\underset{R}{CH}-O)_nH$$

R désignant l'hydrogène ou le groupe méthyle et n un nombre de 1 à 10, ou bien l'éther monométhylique ou monoéthylique d'un tel polyéther ou encore un alcanol en $C_1$ à $C_4$.

2. Procédé selon la revendication 1 pour la préparation de dérivés d'hydroxydiphénylamines de formule (I) ci-dessous

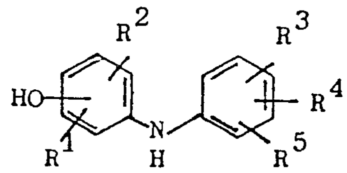

(I)

dans laquelle le groupe —OH peut occuper la position 2, 3 ou 4 et les symboles $R^1$ à $R^5$ désignent chacun, indépendamment les uns des autres, l'hydrogène, un alkyle en $C_1$ à $C_6$ ou un alcoxy en $C_1$ à $C_4$, parmi les substituants $R^3$, $R^4$ et $R^5$, l'un des trois pouvant être également un hydroxyle, deux substituants voisins, parmi $R^1$ et $R^2$ et/ou parmi $R^3$, $R^4$ et $R^5$, peuvent être aussi liés, directement ou par l'intermédiaire d'un hétéro-atome, à un cycle pentagonal à heptagonal condensé et le cycle ainsi formé peut être lui-même substitué par un alkyle en $C_1$ à $C_4$, procédé caractérisé en ce que l'on fait réagir un dérivé de dihydroxybenzène de formule (II)

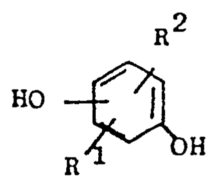

(II)

avec un dérivé d'aniline de formule (III)

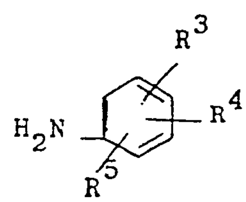

(III)

les divers symboles ayant les significations données ci-dessus.

3. Procédé selon la revendication 1 ou 2, caractérisé en ce que la condensation est effectuée entre 220 et 270 °C.

4. Procédé selon l'une quelconque des revendications 1 à 3, caractérisé en ce que l'on ajoute 0,1 à 1 % en poids d'iode comme catalyseur.

5. Procédé selon l'une quelconque des revendications 1 à 4, caractérisé en ce que, avant de commencer la réaction, on ajoute au mélange des matières dont on part jusqu'à 3 moles d'eau par mole du dérivé de dihydroxybenzène.

6. L'utilisation des dérivés d'hydroxydiphénylamines qui ont été obtenus suivant l'une quelconque des revendications 1 à 5 pour la fabrication de colorants au soufre.